# EUROPEAN PATENT APPLICATION

(11) **EP 2 662 023 A1**
(43) Date of publication of application: **13.11.2013**
(21) Application number: 12732267.5
(22) Date of filing: 05.01.2012
(51) Int. Cl.: A61B 6/03, A61B 6/06, G01T 7/00

(54) **COLLIMATOR AND X-RAY COMPUTED TOMOGRAPHY APPARATUS**

(30) Priority: 07.01.2011 JP 2011002190
(71) Applicant: Kabushiki Kaisha Toshiba, Inc., Tokyo 105-8001 (JP); Toshiba Medical Systems Corporation, Otawara-shi, Tochigi 324-8550 (JP)
(72) Inventor: WATANABE, Kunio, Otawara-shi Tochigi 324-8550 (JP); WAKABAYASHI, Akiji, Otawara-shi Tochigi 324-8550 (JP); SASAKI, Makoto, Otawara-shi Tochigi 324-8550 (JP); NAMBU, Shuya, Otawara-shi Tochigi 324-8550 (JP)
(74) Representative: Granleese, Rhian Jane
(86) International application number: PCT/JP2012/050103
(87) International publication number: WO 2012/093695

(57) **Abstract**

It is an object to improve the collimation performance or focusing performance of a grid collimator with respect to the same point. A collimator includes a collimator frame (16) having a shape corresponding to part of a circular ring, a plurality of first partition plates (31) which are supported on the collimator frame, are radially arrayed along a circumferential direction of the circular ring, and have shielding properties with respect to radiation, a plurality of first guide grooves (35) radially provided in a surface of each of the first partition plates, and a plurality of second partition plates (33) which are supported in the plurality of first guide grooves, are radially arrayed respectively in gaps between the first partition plates, and have shielding properties with respect to the radiation.

## Description

### Technical Field

Embodiments described herein relate generally to a collimator and an X-ray computed tomography apparatus.

### Background Art

The X-ray detector of an X-ray computed tomography apparatus or the like is equipped with a collimator to improve the detectability of direct rays by isolating each detection element from X-rays and removing scattered rays by limiting incident X-ray directions. Recently, a two-dimensional array type X-ray detector has been becoming popular. As this two-dimensional array type X-ray detector, a detector having a relatively small number of detection element rows (also called segments), typically four rows, arranged side by side has been in widespread use. Nowadays, with the use of a solid-state detection element obtained by combining a scintillator and a photodiode element or a solid-state detection element made of selenium or the like which directly converts X-rays into charge, a wide-field X-ray detector having 64 or more detection element rows has appeared on the market. A grid plate obtained by die-cutting a soft-material plate mixed with an X-ray shielding metal powder into a grid pattern is assumed as a collimator applied to such a two-dimensional array type X-ray detector.

In a collimator having such a structure, however, it is impossible to collimate a plurality of collimate regions partitioned by a grid to an X-ray focus.

### Citation List

### Patent Literature

Patent Literature 1: Jpn. Pat. Appln. KOKAI Publication No. 2004-195235
Patent Literature 2: Jpn. Pat. Appln. KOKAI Publication No. 2010-223836
Patent Literature 3: Jpn. Pat. Appln. KOKAI Publication No. 2003-149348
Patent Literature 4: Jpn. Pat. Appln. KOKAI Publication No. 2000-338254
Patent Literature 5: Jpn. Pat. Appln. KOKAI Publication No. 02-253200
Patent Literature 6: Jpn. Pat. Appln. KOKAI Publication No. 2008-89341

### Summary of Invention

### Technical Problem

It is an object to improve the collimation performance or focusing performance of a grid collimator.

### Solution to Problem

A collimator according to this embodiment includes a collimator frame having a shape corresponding to part of a circular ring, a plurality of first partition plates which are supported on the collimator frame, are radially arrayed along a circumferential direction of the circular ring, and have shielding properties with respect to radiation, a plurality of first guide grooves radially provided in a surface of each of the first partition plates, and a plurality of second partition plates which are supported in the plurality of first guide grooves, are radially arrayed respectively in gaps between the first partition plates, and have shielding properties with respect to the radiation.

### Advantageous Effects of Invention

A grid collimator improves its collimation performance or focusing performance.

### Brief Description of Drawings

FIG. 1 is a view showing the outer appearance of a collimator according to this embodiment.
FIG. 2A is a view showing the collimation of a collimate region in FIG. 1.
FIG. 2B is a view showing a collimate line in FIG. 2A.
FIG. 2C is a view showing the arrangement of channel partition plates in FIG. 1.
FIG. 2D is a view the arrangement of slice partition plates in FIG. 1.
FIG. 3 is a view showing a collimator frame in FIG. 1.
FIG. 4 is a view showing the guide grooves of the upper and lower frames of the collimator frame in FIG. 3.
FIG. 5 is a view showing how channel partition plates and slice partition plates are mounted on the collimator frame in FIG. 4.
FIG. 6 is a view showing the direction in which slice partition plates are inserted into channel partition plates in FIG. 5.
FIG. 7A is an enlarged view (plan view) of a portion associated with a grid structure constituted by channel partition plates and slice partition plates in FIG. 1.
FIG. 7B is an enlarged view (perspective view) of a portion associated with a grid structure constituted by channel partition plates and slice partition plates in FIG. 1.
FIG. 8A is a plan view of slice partition plates in FIG. 6.
FIG. 8B is a plan view showing a channel partition plate in FIG. 6.
FIG. 9 is an enlarged view of guide grooves formed in a channel partition plate in FIG. 6.
FIG. 10 is a view showing the collimation of collimate lines in FIG. 9.
FIG. 11 is a partial enlarged view (perspective view) of a grid structure constituted by channel partition plates and slice partition plates in FIG. 1.
FIG. 12 is a view showing the outer appearance of an outer/inner abutment plate of the collimator according to this embodiment.
FIG. 13 is a view showing the overall arrangement of an X-ray computed tomography apparatus including a collimator according to this embodiment.

### Description of Embodiments

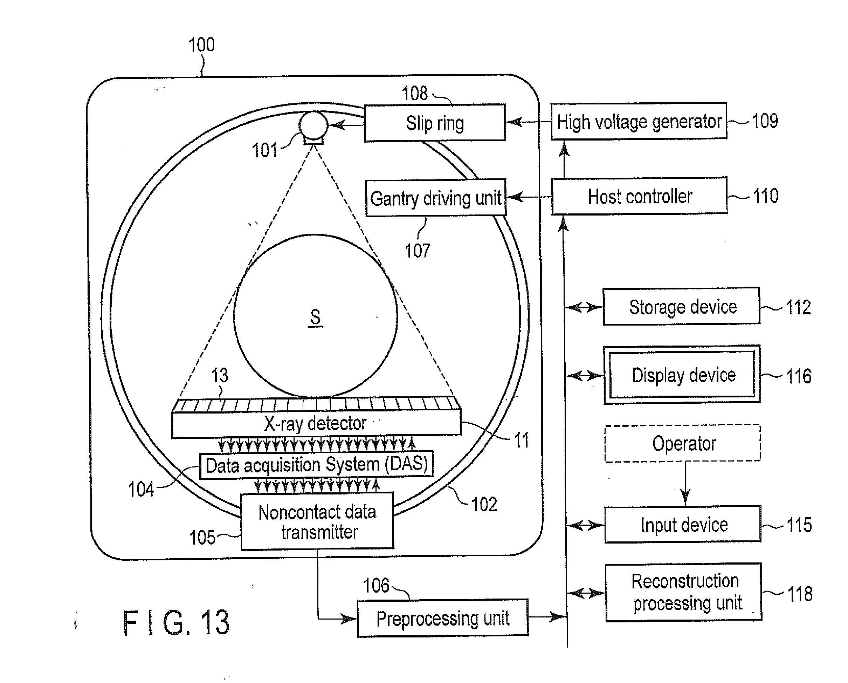

A collimator according to this embodiment will be described below with reference to the accompanying drawings. As shown in FIG. 13, a collimator 13 according to the embodiment includes a single collimator frame 16 having a shape corresponding to part of a circular ring (to be described later). A plurality of channel partition plates 31 are supported on the collimator frame 16. The plurality of channel partition plates 31 are radially arrayed along the circumferential direction of the circular ring of the collimator frame 16. Slice partition plates 33 are radially arrayed in the gaps between the arrayed channel partition plates 31. Each slice partition plate 33 typically has a trapezoidal strip-like shape. Each slice partition plate 33 may have a rectangular strip-like shape. A trapezoidal strip-like shape is superior to a rectangular strip-like shape in terms of focusing performance and scattered ray removal performance. All the slice partition plates 33 have the same shape.

The collimator 13 according to this embodiment is typically mounted on the two-dimensional array type X-ray detector of the X-ray computed tomography apparatus. The X-ray computed tomography apparatus includes a gantry portion (also called a gantry) 100 as a main structural member. The gantry portion 100 includes a rotating ring 102. A cone-beam X-ray tube 101 and an X-ray detector 11 are arranged on the rotating ring 102 so as to face each other. The collimator 13 is mounted on the X-ray detector 11. The collimator 13 will be described in detail later. Upon receiving high-voltage pulses periodically generated from a high voltage generator 109, the X-ray tube 101 generates X-rays. The X-ray detector 11 is formed by an ionization box-type detector or semiconductor detector. If the X-ray detector 11 is a semiconductor X-ray detector, a plurality of X-ray detection elements are arrayed in an arc form centered on the apex (X-ray focus F) of a cone beam, and a plurality of X-ray detection rows are arranged side by side in a direction almost parallel to the rotation axis of the rotating ring 102. A data acquisition system 104 generally called a DAS (Data Acquisition System) is connected to the X-ray detector 11. The data acquisition system 104 is provided with, for each channel, an I-V converter for converting the current signal obtained via each channel of the X-ray detector 11 into a voltage, an integrator for periodically integrating these voltage signals in synchronism with an X-ray irradiation period, an amplifier for amplifying an output signal from the integrator, and an analog/digital converter for converting an output signal from the amplifier into a digital signal. A preprocessing unit 106 is connected to the data acquisition system 104 via a noncontact data transmitter 105. The preprocessing unit 106 performs preprocessing, for the projection data detected by the data acquisition system 104, such as sensitivity unevenness correction processing between channels and the processing of correcting an extreme decrease in signal intensity or signal omission due to an X-ray absorber, mainly a metal portion. A storage device 112 stores projection data corrected by the preprocessing unit 106. A reconstruction processing unit 118 reconstructs volume data representing a three-dimensional distribution of CT values by an arbitrary cone beam image reconstruction algorithm based on stored projection data. A typical example of this cone beam image reconstruction algorithm is the weighted Feldkamp method. The Feldkamp method is an approximate reconstruction method based on a fan beam convolution/back projection method. Convolution processing is performed by regarding data as fan projection data on the premise that the cone angle is relatively small. However, back projection processing is performed along an actual ray.

An image processing unit (not shown) converts volume data into image data expressed by a two-dimensional coordinate system by rendering processing, multi-planar reformatting (MPR), or the like. A display device 116 displays image data. A host controller 110 controls a gantry driving unit 107 to stably rotate the rotating ring 102 at a constant speed in order to acquire projection data, that is, execute scanning. The host controller 110 performs overall control associated with scanning, for example, controlling the high voltage generator 109 to generate X-rays from the X-ray tube 101 during a scanning period, and controlling the data acquisition system 104 or the like in synchronism with X-ray generation.

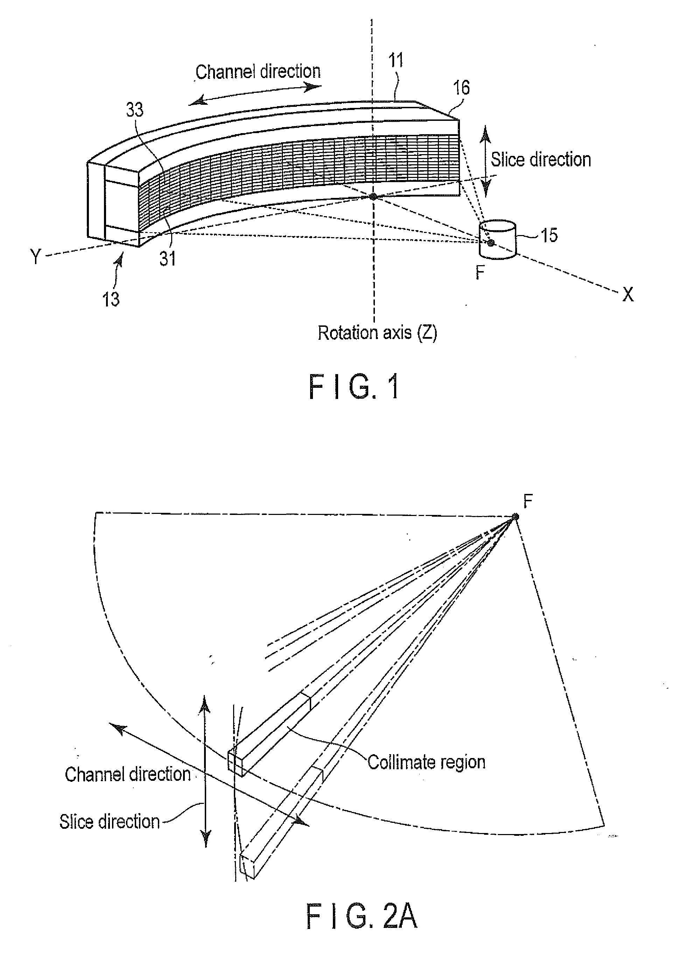

As shown in FIG. 1, the two-dimensional array type X-ray detector 11 typically has a plurality of X-ray detection elements arrayed in a row in an arc form centered on the X-ray focus, and a plurality of X-ray detection rows are arranged side by side in the Z-axis direction (slice direction). Note that the array direction of the detection elements within an X-ray detection element row is called a channel direction.

The collimator 13 in which X-ray shielding plates (partition plates) are assembled in a grid form in two directions, that is, the slice and channel directions, is provided on the X-ray source side of the two-dimensional array type X-ray detector 11 to improve the detectability of direct rays by optically isolating the respective detection elements and removing scattered rays by limiting incident directions.

In this case, a conventional collimator includes a plurality of collimator modules having the same structure. The plurality of collimator modules are arrayed in a polygonal shape centered on the X-ray focus. Each module covers part of a matrix (assumed to have n x m channels). Let m be the number of channels in the slice direction, and n be the number obtained by dividing a total number N of channels in the slice direction in the X-ray detector 11 by the number of collimator modules. Each module has (n + 1) flat channel partition plates for isolating the detection elements in the channel direction. The (n + 1) channel partition plates are arrayed on the module frame at slightly different mounting angles along the channel direction. In addition, each module includes (m + 1) slice partition plates for isolating the detection elements in the slice direction. Each slice partition plate has a comb-like shape with a width that covers a sensitivity width corresponding to n channels. These slice partition plates are commonly inserted in the (n + 1) channel partition plates. Although the central channel of the (n x m) collimate regions surrounded by the channel partition plates and the slice partition plates is collimated to the X-ray focus, the collimations of many other collimate regions deviate from the X-ray focus. This problem becomes more pronounced with an increase in the number of channels in the slice direction. In addition, sensitivity deteriorates at the joint portions of the collimator modules. Furthermore, stress due to centrifugal force accompanying high-speed rotation concentrates on the joint portion of each collimator module. This may lead to an uneven sensitivity distribution. This embodiment can solve these problems.

The collimator 13 can be fabricated as an integral structure as a whole in accordance with the fan angle of X-rays unlike the prior art in which a plurality of modules, each assembled independently, are arrayed in an arc form.' In addition, the collimator 13 implements a structure which allows to have predetermined curvatures in the two directions, that is, the channel and slice directions, so as to accurately collimate all the collimate regions to the X-ray focus F. Note that the collimate regions are those that are surrounded by vertical and horizontal partition plates constituting the collimator 13. A line connecting the center of one end face of each collimate region to the center of the other end face is called a collimate line. The collimate line indicates the directivity of the collimate region.

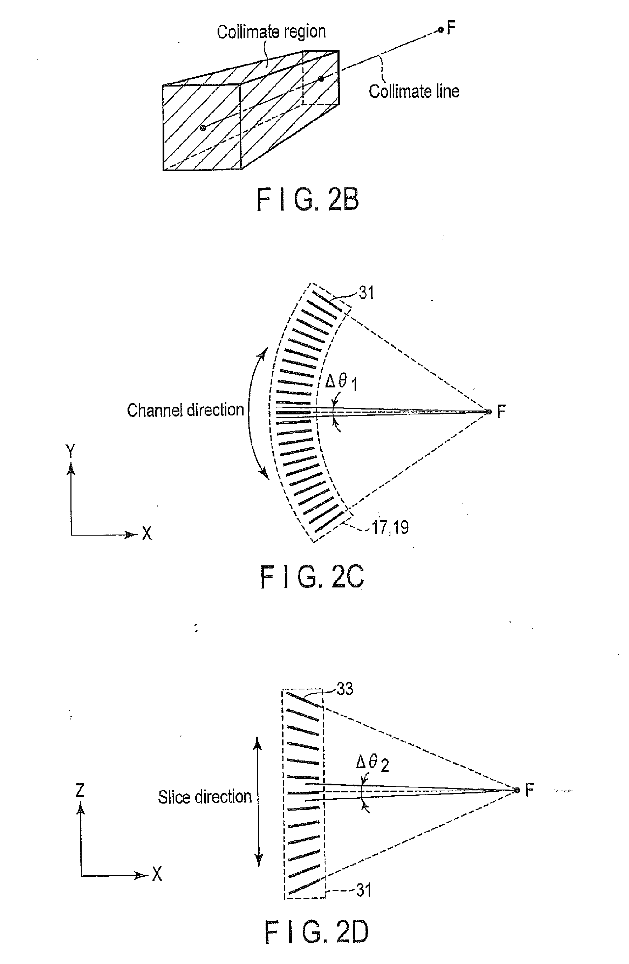

As shown in FIGS. 2A, 2B, 2C, and 2D, the collimator 13 includes, as main constituent elements, the plurality of channel partition plates 31 and the plurality of slice partition plates 33 which have shielding properties against radiation, X-rays in this case. The channel partition plates 31 and the slice partition plates 33 are formed from thin plates having X-ray shielding properties. The channel partition plates 31 optically isolate the detection elements of the X-ray detector 11 in the channel direction. The slice partition plates 33 optically isolate the detection elements of the X-ray detector 11 in the slice direction. The channel partition plates 31 and the slice partition plates 33 are assembled in a grid form.

The plurality of channel partition plates 31 partition a plurality of collimate regions, together with the plurality of slice partition plates 33. The plurality of collimate regions respectively correspond to the plurality of detection elements. The plurality of channel partition plates 31 and the plurality of slice partition plates 33 are assembled so as to collimate the plurality of collimate regions to one point, the X-ray focus F in this case.

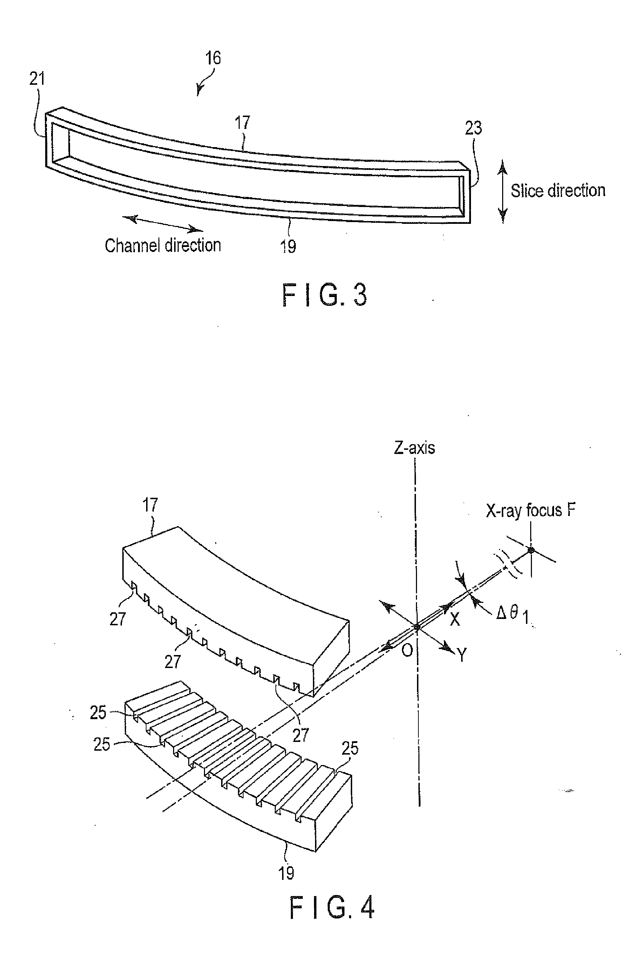

The collimator 13 includes the collimator frame 16. As shown in FIG. 3, the collimator frame 16 includes an upper frame 17 and a lower frame 19. The upper frame 17 has a shape corresponding to part of a circular ring plate. The lower frame 19 has the same shape as that of the upper frame 17. The radius of the circular ring of the upper frame 17 or lower frame 19 is almost equal to the distance between the collimator frame 16 attached to the X-ray detector 11 and mounted on the rotating ring 102 and the X-ray focus F of the cone-beam X-ray tube 101. The upper frame 17 and the lower frame 19 are held parallel by side frames 21 and 23. The interval between the upper frame 17 and the lower frame 19 is almost equal to the length of a channel partition plate guide groove (to be described later), more specifically, the sensitivity width of the X-ray detector 11 in the slice direction.

As shown in FIG. 4, a plurality of guide grooves (to be referred to as channel partition plate guide grooves) 25 are formed in the lower frame 19. The plurality of channel partition plate guide grooves 25 are radially formed at predetermined intervals in the channel direction centered on the X-ray focus F. In other words, the plurality of channel partition plate guide grooves 25 are formed along the radial direction of a circular ring centered on the X-ray focus F. As shown in FIG. 2C, the plurality of channel partition plate guide grooves 25 are regularly arrayed such that a spread angle Δθ1 defined as the angle between adjacent collimate lines in the channel direction becomes constant. Likewise, channel partition plate guide grooves 27 are formed in the upper frame 17.

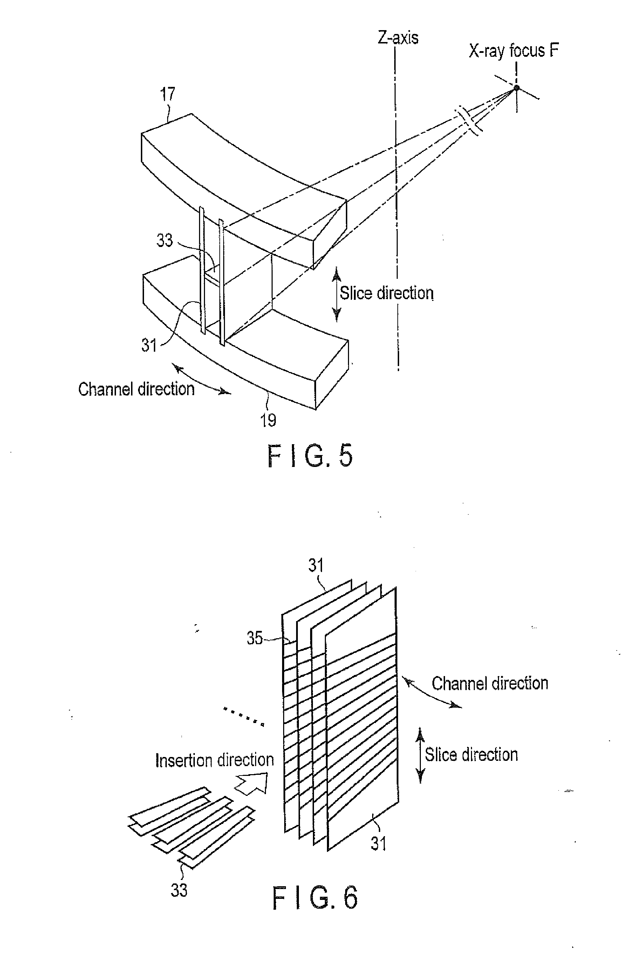

As shown in FIG. 5, the plurality of channel partition plates 31 are supported in the plurality of channel partition plate guide grooves 25 and are fixed with an epoxy-based adhesive. The channel partition plates 31 are provided perpendicular to the frames 17 and 19. As shown in FIG. 2C, the plurality of channel partition plates 31 are radially arrayed at predetermined intervals in the channel direction centered on the X-ray focus F in accordance with the plurality of channel partition plate guide grooves 25. In other words, the channel partition plates 31 are arranged along the radial direction of a circular ring centered on the X-ray focus F.

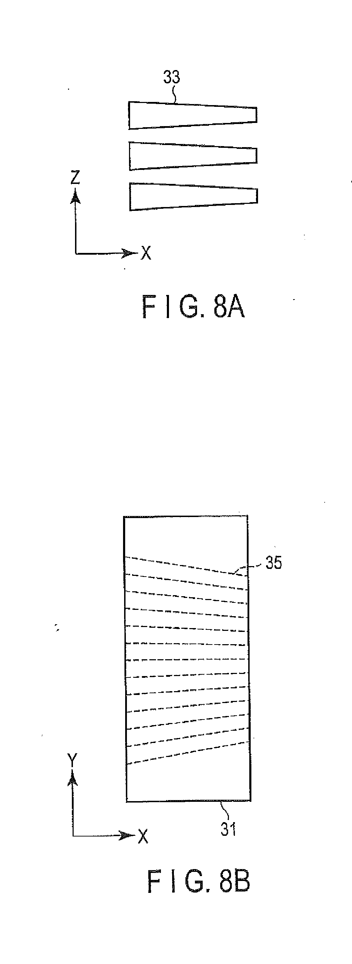

As shown in FIG. 8B, the channel partition plate 31 is a rectangular thin plate having shielding properties. A plurality of slice partition plate guide grooves 35 are formed in the upper and lower surfaces of each channel partition plate 31. The plurality of slice partition plate guide grooves 35 have almost the same shape and size. The plurality of slice partition plate guide grooves 35 are radially arrayed at predetermined intervals in the slice direction centered on the X-ray focus F. As shown in FIG. 2D, a spread angle Δθ2 defined as the angle between adjacent collimate lines in the slice direction is set constant.

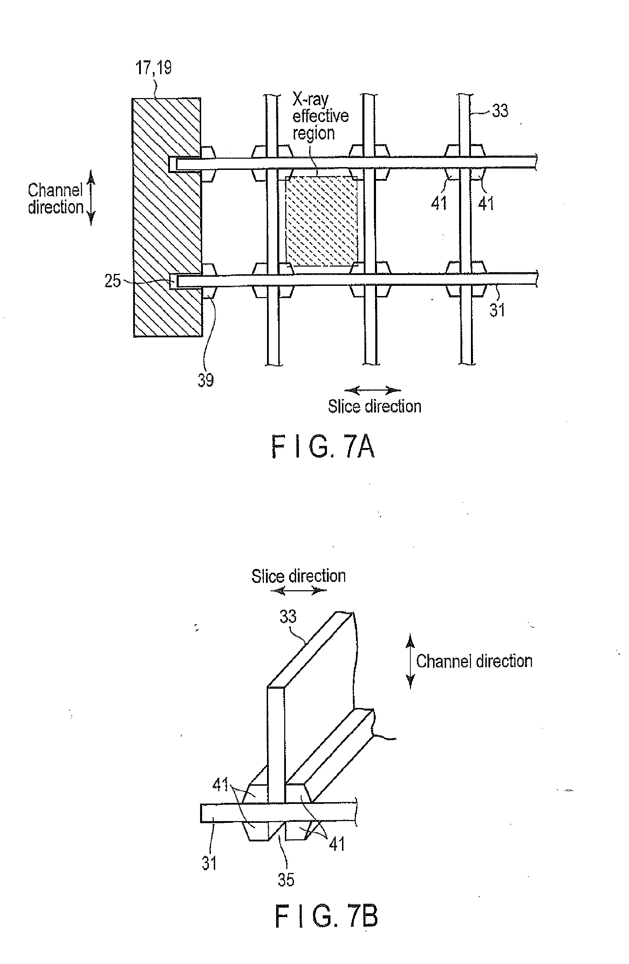

As shown in FIGS. 7A, 7B, 8B, and 9, the slice partition plate guide groove 35 is constituted by a pair of guide rail 41 made of a resin or metal and directly formed on the surface of the channel partition plate 31. The slice partition plate guide groove 35 may be cut in the surface of the channel partition plate 31. The following description will be made with reference to the former case. The rails at the farthest ends are used as stopper rails 39 for the frames 17 and 19, as shown in FIG. 7A.

As shown in FIG. 8A, the slice partition plate 33 is a thin plate having a trapezoidal strip-like shape, which is made of a material having X-ray shielding properties. All the slice partition plates 33 have the same shape and size.

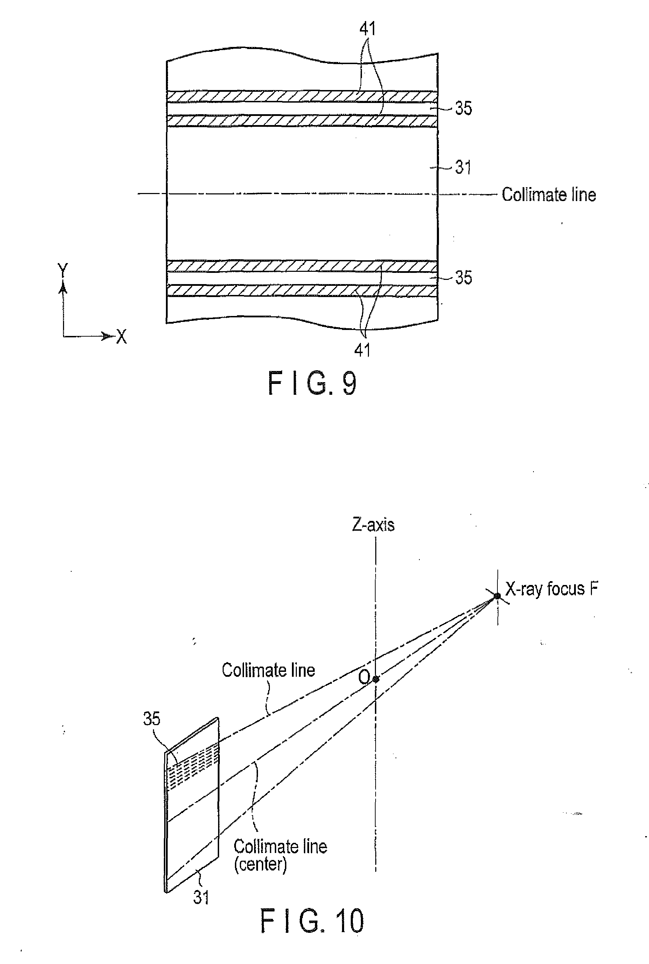

As shown in FIG. 6, the plurality of slice partition plates 33 are supported in the slice partition plate guide grooves 35 in the gaps between the adjacent channel partition plates 31, and are fixed with an epoxy-based adhesive or by laser welding. The slice partition plates 33 are provided perpendicular to the channel partition plates 31. As shown in FIGS. 2D and 10, the plurality of slice partition plates 33 are radially arrayed at predetermined intervals in the slice direction centered on the X-ray focus F in accordance with the plurality of slice partition plate guide grooves 35.

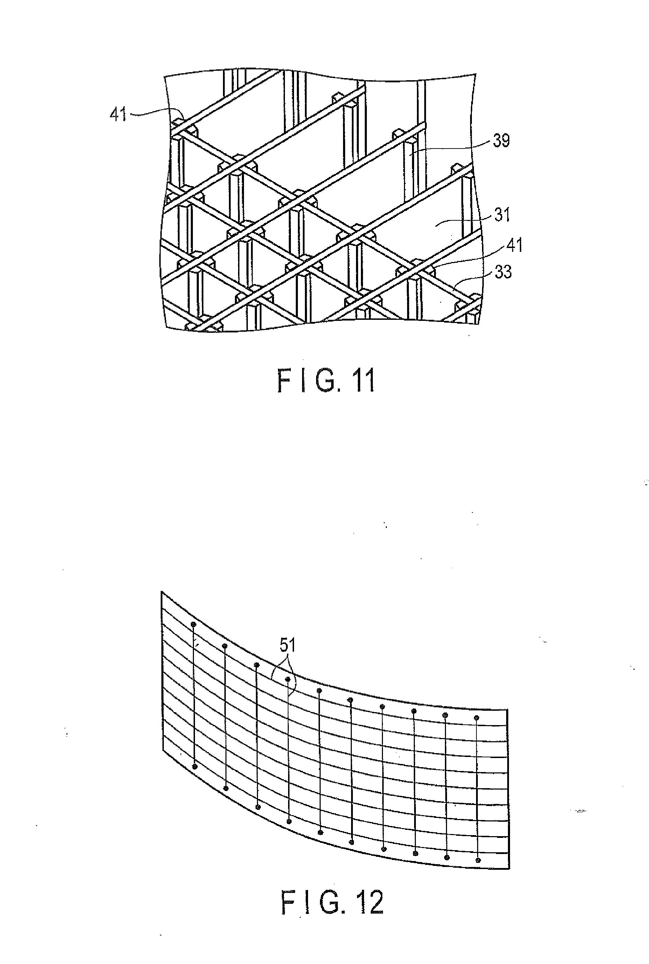

As shown in FIG. 11, the collimator 13 includes the plurality of channel partition plates 31 and the plurality of slice partition plates 33 which are assembled in a grid form. The channel partition plates 31 are radially arrayed at predetermined angular intervals Δθ1 along the channel direction (see FIG. 2C). The plurality of slice partition plates 33 are arranged in the gaps between the channel partition plates 31. The plurality of slice partition plates 33 are radially arrayed at predetermined angular intervals Δθ2 along the slice direction (see FIG. 2D). The plurality of channel partition plates 31 and the plurality of slice partition plates 33 partition a plurality of pyramidal collimate regions. A plurality of collimate lines respectively corresponding to the plurality of collimate regions focus to one point (X-ray focus F). This focusing property greatly reduces scattered rays entering a plurality of channels and greatly improves diagnostic image accuracy.

This focusing property can be achieved by the assembly of the channel partition plates 31 and the slice partition plates 33. In other words, since the channel partition plates 31 and the slice partition plates 33 eliminate the necessity of mechanical processing such as curving, bending, twisting, and welding, it is possible to solve the problems of strength reduction and stress concentration due to such mechanical processing. This can also greatly reduce distortion, deflection, and tortion. In addition, the above focusing property can be obtained by the integral arrangement of the collimator 13 instead of the assembly of a plurality of modules. The collimator 13 is free from problems such as partial distortion due to excessive stress on module joint portions and the like and a great change in scattered ray removal accuracy at module joint portions, and can obtain continuity of output characteristics in the two directions, that is, the channel and slice directions.

The channel partition plates 31 and the slice partition plates 33 assembled in a grid form are internally and externally supported by the inner and outer abutment plates. The inner and outer abutment plates guarantee the maintenance of the grid form of the channel partition plates 31 and the slice partition plates 33. FIG. 12 shows the outer appearance of the inner abutment plate. The outer abutment plate has a shape similar to that of the inner abutment plate. Abutment plates are formed from curved thin plates made of, for example, acrylic resin having X-ray transmittance. Groove portions 51 which receive the channel partition plates 31 and the slice partition plates 33 are vertically and horizontally formed in the outer surface of the inner abutment plate. The channel partition plates 31 and the slice partition plates 33 are fitted in the groove portions 51. Likewise, groove portions which receive the channel partition plates 31 and the slice partition plates 33 are vertically and horizontally formed in the inner surface of the outer abutment plate. The abutment plates may be removed in the process of assembly. The completed collimator 13 may not be equipped with the abutment plates.

The channel partition plate 31 is formed by cutting a thin molybdenum original plate having X-ray shielding properties into a rectangle. Guide rails are formed on the surface of the molybdenum plate with a resin. The slice partition plate 33 is molded from, for example, a thin molybdenum original plate having X-ray shielding properties.

The inner abutment plate is fixed to the inner side of the collimator frame 16 with an adhesive and by thread fastening. The channel partition plates 31 are inserted in the channel partition plate guide grooves 25, are made to abut against the inner abutment plate, and are fixed with, for example, an adhesive. The slice partition plates 33 are inserted in the slice partition plate guide grooves 35 of the channel partition plates 31 fixed to the collimator frame 16, and are made to abut against the outer abutment plate. The outer abutment plate is fixed to the collimator frame 16 by thread fastening.

Some embodiments of the present invention have been described above. However, these embodiments are presented merely as examples and are not intended to restrict the scope of the invention. These embodiments can be carried out in various other forms, and various omissions, replacements, and alterations can be made without departing from the gist of the invention. The embodiments and their modifications are also included in the scope and the gist of the invention as well as in the invention described in the claims and their equivalents.

### Reference Signs List

11... X-ray detector
13...collimator
15...X-ray tube
31...channel partition plate
33...slice partition plate

## Claims

1. A collimator comprising:
a collimator frame having a shape corresponding to part of a circular ring;
a plurality of first partition plates which are supported on the collimator frame, are radially arrayed along a circumferential direction of the circular ring, and have shielding properties with respect to radiation;
a plurality of first guide grooves radially provided in a surface of each of the first partition plates; and
a plurality of second partition plates which are supported in the plurality of first guide grooves, are radially arrayed respectively in gaps between the first partition plates, and have shielding properties with respect to the radiation.

2. The collimator of claim 1, wherein both the first partition plate and the second partition plate form a plurality of collimate regions, and
a plurality of collimate lines corresponding to the plurality of collimate regions focus to one point.

3. The collimator of claim 2, wherein the collimate region has a pyramidal shape.

4. The collimator of claim 1, wherein the second partition plate has a strip-like shape.

5. The collimator of claim 1, wherein the second partition plate has a trapezoidal strip-like shape.

6. The collimator of claim 1, wherein all the second partition plates have the same shape.

7. The collimator of claim 1, wherein the first guide groove is provided by guide rails formed on a surface of the first partition plate.

8. The collimator of claim 1, wherein the first guide groove is formed by cutting in the surface of the first partition plate.

9. The collimator of claim 1, wherein the second partition plate is fixed to the first partition plate with an epoxy-based adhesive.

10. The collimator of claim 1, further comprising a plurality of second guide grooves radially provided in a surface of the collimator frame,
wherein the first partition plate is supported in the second guide groove.

11. The collimator of claim 10, wherein the first partition plate is fixed to the collimator frame with an epoxy-based adhesive.

12. The collimator of claim 1, wherein the first partition plates and the second partition plates are arrayed at predetermined angular intervals.

13. The collimator of claim 1, wherein the first partition plates have the same shape.

14. The collimator of claim 1, further comprising an inner abutment plate provided inside the collimator frame and configured to align the first partition plates and the second first partition plates.

15. The collimator of claim 14, further comprising an outer abutment plate provided outside the collimator frame and configured to align the first partition plates and the second first partition plates.

16. The collimator of claim 15, wherein groove portions which receive the first partition plates and the second partition plates are formed in the inner abutment plate and the outer abutment plate.

17. An X-ray computed tomography apparatus comprising:
an X-ray source;
an X-ray detector including a plurality of X-ray detection elements which detect X-rays generated from an X-ray focus of the X-ray source and transmitted through an object to be examined, the plurality of X-ray detection elements being arrayed in a channel direction and a slice direction;
a collimator mounted on the X-ray detector; and
a reconstruction unit which reconstructs image data associated with the object based on an output from the X-ray detector,
wherein the collimator comprises
a collimator frame having a shape corresponding to part of a circular ring,
a plurality of first partition plates which are supported on the collimator frame, are radially arrayed along a circumferential direction of the circular ring, and have shielding properties with respect to radiation,
a plurality of first guide grooves radially provided in a surface of each of the first partition plates, and
a plurality of second partition plates which are supported in the plurality of first guide grooves, are radially arrayed respectively in gaps between the first partition plates, and have shielding properties with respect to the radiation

18. The X-ray computed tomography apparatus of claim 17, wherein both the first partition plate and the second partition plate are provided with a plurality of collimate regions, and
a plurality of collimate lines corresponding to the plurality of collimate regions focus to one point.

19. The X-ray computed tomography apparatus of claim 17, wherein the second partition plate has a trapezoidal strip-like shape.

20. The X-ray computed tomography apparatus of claim 17, wherein the first guide groove is provided by guide rails formed on a surface of the first partition plate.
